# EUROPEAN PATENT SPECIFICATION

(11) **EP 1 804 850 B1**
(45) Date of publication and mention of the grant of the patent: **11.03.2009**
(21) Application number: 05819307.9
(22) Date of filing: 28.10.2005
(51) Int. Cl.: A61L 31/12, A61L 31/06, A61L 27/40

(54) **BIOABSORBABLE POLYMERS COMPRISING CALCIUM CARBONATE**
BIOABSORBIERBARE POLYMERE MIT KALZIUMKARBONAT
POLYMERES BIOABSORBABLES COMPRENANT DU CARBONATE DE CALCIUM

(30) Priority: 29.10.2004 US 623645 P
(43) Date of publication of application: 11.07.2007
(73) Proprietor: Smith and Nephew, Inc., Memphis, TN 38116 (US)
(72) Inventor: COTTON, Nicholas, John, Westborough, MA 01581 (US); BRUNELLE, John, Eric, Newport Coast, CA 92657 (US)
(74) Representative: Connors, Martin L.H.
(86) International application number: PCT/US2005/038977
(87) International publication number: WO 2006/050119

(56) References cited:
- WO-A-00/30552
- WO-A-01/66044
- WO-A-20/05018700
- WO-A-20/05061617
- FR-A- 2 364 644
- US-A- 5 433 751
- US-A- 5 741 329
- US-A1- 2003 129 401
- US-B1- 6 344 496

## Description

### TECHNICAL FIELD

This invention relates to compositions that include a biodegradable copolymer such as poly(lactide-co-glycolide) (PLGA) and to methods of making and using devices containing such compositions.

### BACKGROUND

Tissue fixation devices are used extensively to repair traumatic injuries, including those sustained during sporting events. Many of these devices are used to reattach soft tissue to bone. For example, interference screws are used to fixate autologous grafts during anterior cruciate ligament (ACL) repair. The devices are often made from a semi-crystalline polymer, poly(1-lactic acid) (PLLA) or copolymers of PLLA with poly(dl-lactic) acid (PDLA) or poly(glycolic) acid (PGA). These bioabsorbable polymers produce acidic products upon degradation, and others have suggested inclusion of a buffering compound to neutralize the breakdown products (*see, e.g.,* U.S. Patent No. 6,741,329). Although appropriate for soft tissue repair, these materials can also be used in the event of orthopedic trauma or reconstructive surgery to fixate bone to bone.

US5741329 refers to a biocompatible composition comprising a copolymer formed from lactic acid monomers and glycolic acid monomers, wherein the copolymer is poly(lactide-co-glycolide) in a ration of 50:50 and a calcium carbonate in an amount of 1-99 wt%. The biocompatible composition comprises also a therapeutic agent and is used as internal fixation device such as screws.

US6344496 refers to a biodegradable implant comprising a poly(lactide-co-glycolide) copolymer (85:15) and a bioactive ceramic in order to provide a biodegradable polymeric therapeutic implant materials.

US5433751 describes a bioresorbable bone prosthesis material containing particles of calcium carbonate in an amount of 40-70 wt% dispersed within a polymer matrix, e.g. lactic acid/glycolic acid copolymer.

US2003/129401 refers to compositions based on copolymers and calcium carbonate.

WO00/30552 describes a surgical device such as a screw for bones comprising a copolymer or polymer which is bioabsorbable and comprises particulate or fibrous solid material made of calcium carbonate.

WO01/66044 describes a shaped particle composite for use in treating bone deficiency.

WO2005/018700 and WO2005/061617 disclose a kit comprising a copolymer and calcium carbonate.

### SUMMARY

The invention described below is based, in part, on studies we conducted with biocompatible compositions that contain a copolymer and a filler material. Accordingly, the invention features compositions that include a copolymer. The compositions include a copolymer that includes lactic acid and glycolic acid monomers and a filler *i.e.* calcium carbonate as described in claim 1.

In specific embodiments, the copolymer can be poly(lactide-co-glycolide) (PLGA), with a lactide:glycolide ratio of about 85:15 and the filler can be calcium carbonate. We may refer to compositions containing calcium carbonate as Poly Lactide Carbonate or "PLC." More generally, we refer to "compositions" in describing a certain aspect of our invention, but we may also use the terms "materials" or "biomaterials" or, when the compositions are fashioned for a particular use, such as implantation, to "devices" or "implants." Further, where the devices are suitable for attaching one tissue to another (*e.g*., attaching soft tissue to bone or attaching bone to bone), we may refer to them as internal fixation devices. Such devices include screws, pins, rods, plates, sutures, suture anchors, staples, clips, rings, and the like. When fashioned to repair an injured bone (*e.g*., when used to replace lost bone fragments), the device can be described as a bone prosthesis.

The compositions of the invention can be amorphous (*i.e.*, they can be compositions in which the polymer chains are not ordered) or semi-crystalline (*i.e.*, compositions in which there is some order to the polymer chains). On a macroscopic level, the compositions can have a pulverized or pelletized form (for example, the compositions of the invention can be formulated as a powder or paste, or as pellets, granules, or interlocking shapes), or they can be shaped for use in a particular surgical procedure (for example, as a tissue fixation device or synthetic bone substitute or prosthesis). In any event, the compositions can be sterile. The compositions can also be fashioned as porous implants or devices. Methods for making such implants or devices are known in the art and can be carried out with the compositions of the present invention. For example, processes are known in the art for using porogens, leaching agents, supercritical CO₂, gas generating additives, and/or sintering techniques to fuse smaller shapes. The compositions of the invention can also be molded into essentially any shape, whether regular (such as a cylinder or square) or irregular.

The compositions of the invention are useful in a wide variety of methods in which tissue is altered, including methods in which the primary site of repair is bone *per se.* The methods encompass any type of tissue modification, including tissue repair, reconstruction, remodeling, and tissue-guided regeneration. In addition to their use as tissue fixation devices or synthetic bone substitutes or prostheses, the compositions of the invention can be used as devices for attachment of orthopedic hardware (*e.g*., as screws for bone plates or screws to temporary secure hip stems) or in the context of reconstructive or cosmetic surgery.

The invention features a biocompatible (*i.e.*, substantially non-toxic) composition that includes a filler, *i.e.* calcium carbonate and a copolymer formed from lactic acid monomers and glycolic acid monomers. The filler calcium carbonate constitute more than 30% but less than 40% of the weight of the composition, regardless of the composition's form, the copolymer selected, or the inclusion of other components (*e.g*., a therapeutic agent, as described below). For example, the filler, *i.e.* calcium carbonate can constitute more than 30% but less than about 34%; more than 30% but less than about 35%; or about 36% to less than 40% of the weight of the composition. The filler can constitute more than 30%; about 31%; about 32%; about 33%; about 34%; about 35%; about 36%; about 37%; about 38%; about 39%; or an amount therein between (*e.g*., an amount between 31 and 32%; an amount between 32 and 33%; and so forth). Where calcium carbonate is used, it can have the crystalline structure of calcite, and it may be present as calcium carbonate particles of a substantially uniform size (*e.g*., a majority of the calcium carbonate particles can be 0.1-0.5; 0.5-2.5; 2.5-5.0; 5.0-7.5; or 7.5-10.0 µm in size (size being measured across the particles' largest diameter)). Alternatively, the filler particles can vary in size (*e.g*., ranging in size in a uniform or non-uniform way from 0.01 µm to 10.0 µm).

Any of the fillers, including CaCO₃, can be combined with a PLGA copolymer in which the lactic acid monomers are in the L-form or the D-form, or are a mixture of the L- and D-forms. More specifically, the copolymer can be poly(dl-lactide-co-glycolide). The ratio of lactic acid and glycolic acid monomers within the polymer can also vary. For example, the copolymer can contain from 50:50 lactide:glycolide units to 90:10 lactide:glycolide units (*e.g*., 85:15 lactide:glycolide units). It will be understood by one of ordinary skill in the art that these ratios can, and often do, vary due to manufacturing limitations. For example, the ratio can vary by about ± 5%. Thus, it is to be understood that all references herein to the ratio of polymer units encompasses copolymers in which that ratio varies to an expected extent. In a specific embodiment, the composition includes (and may include only) a copolymer of lactide and glycolide units and more than 30% but less than 40% calcium carbonate by weight. In another specific embodiment, the composition includes (and may include only) poly(lactide-co-glycolide) at 85:15 lactide:glycolide units and more than 30% but less than 40% of calcium carbonate by weight (*e.g*., 30-34%, 35%, or 36-40%). Regardless of the precise components or their amounts, the copolymer can be amorphous or crystalline and the filler (*e.g*. CaCO₃) and the copolymer (*e.g*., PLGA) can form a substantially homogeneous mixture (*e.g*., the filler can be evenly or about evenly distributed within the copolymer; dispersed). Thus, the composition of any device, as a whole, fashioned from a substantially homogeneous mixture can also be homogeneous (*e.g*., the composition of a device at the proximal and distal ends of a screw or the opposite faces of a plate can be substantially indistinguishable in content).

The compositions described herein can, but do not necessarily, contain one or more additional components, which may be bioactive agents (*e.g*., therapeutic agents). For example, the compositions can contain a growth factor, including growth factors such as those from the fibroblast growth factor family, transforming growth factor family, or platelet derived growth factor family that act as chemoattractants and/or growth stimulators, a hormone such as human growth hormone, an antibiotic, an antiviral agent, an antifungal agent, an anti-inflammatory agent, an inflammatory mediator such as an interleukin, tumour necrosis factor, a prostaglandin, nitric oxide, an analgesic agent, an osteogenic factor such as a bone morphogenetic protein, or a matrix molecule such as hyaluronan. Other agents include angiogenic factors, which are capable of directly or indirectly promoting angiogenesis. Examples include angiogenic peptide growth factors in autologous, xenogenic, recombinant, or synthetic forms (*e.g*., a member of the vascular endothelial growth factor family). Further examples are blood clot breakdown products, such as thrombin and heparin including autologous, allogeneic, xenogeneic, recombinant and synthetic forms of these materials. Compositions based around butyric acid, including butyric acid (butanoic acid, C₄H₈O₂) and butyric acid salts, including sodium, potassium, calcium, ammonium and lithium salts, α-monobutyrin (1-glycerol butyrate; 1-(2,3 dihydroxypropyl) butanoate; C₇H₁₄O₄) and hydroxybutyrate can also be incorporated. Where the bioactive or therapeutic agent is a polypeptide, one can incorporate the polypeptide in its naturally occurring form or a fragment or other mutant thereof that retains sufficient biological activity to confer a benefit on the patient to whom it is administered. The polypeptides can be autologous in the sense that, where the recipient is a human patient, the polypeptide can have the sequence of a human polypeptide or a biologically active fragment or other mutant thereof. Alternatively, or in addition, the additional component can be a nutraceutical, such as a vitamin or mineral.

The bioactive material is included in an amount that is therapeutically effective for the organism (*e.g*., a human patient) in question. Inclusion of one or more bioactive materials may, for example, increase the rate of tissue repair, decrease the risk of infection, or otherwise aid the healing or post-operative process.

In another aspect, the invention features methods of making devices (*e.g*., internal fixation devices) with the compositions described herein. In one embodiment, the method can be carried out in steps that include the following: (a) providing a filler (*e.g*., calcium carbonate); (b) providing a copolymer (*e.g*. a copolymer formed from lactic acid monomers and glycolic acid monomers); (c) combining the filler and the copolymer to produce a composition in which the amount of the filler constitutes more than 30% and less than 40% of the composition (*e.g*., about 35%); and (d) molding the composition to produce a device (*e.g*., an internal fixation device). In a specific embodiment, the method will produce a composition that includes (and may include only) a copolymer of lactide and glycolide units and more than 30% but less than 40% calcium carbonate by weight. In another specific embodiment, the method will produce a composition that includes (and may include only) poly(lactide-co-glycolide) at 85:15 lactide and glycolide units and 20-50% calcium carbonate by weight (*e.g*., 20-30%, 30-40%, 40-50%, 30-34%, 35%, or 36-40%). The methods can further include a step of sterilizing the device by, for example, exposing it to radiation (*e.g*., gamma radiation), treating it with gases (*e.g*., chemical sterilization such as exposure to ethylene oxide gas), exposing it to heat (*e.g*., from steam, as in autoclaving), or exposing it to an electronic beam (e beam), or light (*e.g*., white light). Methods of sterilizing devices are known in the art, and one of ordinary skill in the art can select methods appropriate for a given device.

Optionally, the filler and copolymer can be combined with a bioactive agent (*e.g*., a therapeutic agent) including, but not limited to, any of those described herein. The therapeutic agent can be mixed or otherwise combined with the copolymer and filler or it can be added to the surface of the device or otherwise localized within the device.

If desired, one can omit the molding process of step (d). Thus, the methods of the invention encompass those comprising steps (a)-(c) above, but not step (d). Therapeutic agents can also be included, and the composition can be sterilized and packaged, just as molded compositions can be sterilized and packaged. Thus, the invention includes internal fixation devices (specific and non-limiting examples of which are included herein) that include, or that consist of, a biocompatible composition described herein (*e.g*., a screw, pin, plate, nail, or suture anchor).

The materials within the composition or device can be combined by any method that produces a satisfactory mixture that can be, if desired, formed into a shaped device. For example, a device can be formed by an extrusion process (*e.g*., a single screw, twin screw, disk, ram, or pulltrusion process); a molding process (*e.g*., an injection, intrusion, compression, or thermoforming process); a solvent based process (*e.g*., mixing or casting); a welding process (*e.g*., an ultrasonic or hermetic process); a polymerization process (*e.g*., reaction injection molding, bulk polymerization, and solvent polymerization); or by other methods (*e.g*., fiber spinning or electrospinning). The components within the compositions or devices can have the properties described herein. For example, where the filler is calcium carbonate, it can have the particle size described above, the lactic acid monomers used can be in the D-form, L-form, or a mixture ofD- and L-forms, and so forth.

The compositions or devices can be packaged as kits, with instructions for further processing them or using (*e.g*., implanting) them. The instructions can be, but are not necessarily, printed instructions (*e.g*., the instructions can be supplied as an audio- or videotape or on a compact disc or similar medium). The kits can optionally contain materials suitable for processing or using the compositions or devices.

In another aspect, the invention features methods of using the compositions and devices to repair or remodel tissue. For example, the compositions and devices can be used in treating a patient who has sustained an injury in which a soft tissue within their body has become detached (wholly or partly) from bone. The methods can be carried out by using an internal fixation device as described herein (or made according to the methods described herein) to reattach the soft tissue to the bone. The soft tissue can be a ligament, (*e.g*., the ACL), a tendon, a muscle, cartilage, or other soft or connective tissue. In other embodiments, the compositions and devices of the invention can be used to repair or reshape a bone or to attach bone to bone. The invention thus features use of a composition or device as described herein for the repair or remodeling of tissue (*e.g*., of a bone defect or a defect manifest as a tear or dissociation of a soft tissue, as described above, from bone). The invention further features use of a composition as described herein (*e.g*., a biocompatible composition as described above and further below) for the preparation of a medicament for the repair or remodeling of tissue.

The invention also features methods of treating a patient who has, or who is at risk for developing, osteomyelitis (an acute or chronic bone infection, usually caused by bacteria, and frequently associated with trauma, diabetes, and any condition associated with frequent disruption of the skin (*e.g*., hemodialysis, intravenous therapy, and drug abuse)). The method can be carried out by administering to the patient a composition or device described herein that includes an antibiotic. For example, where a patient has developed osteomyelitis in connection with a traumatic injury, the injury can be repaired with a suitable device that includes an antibiotic. The invention thus features use of a composition or device as described herein (*e.g*., a composition that includes and may include only, a filler (*e.g*., calcium carbonate) and a copolymer (*e.g*., a copolymer formed from lactic acid monomers and glycolic acid monomers) and an antibiotic for the treatment of osteomyelitis. As noted, the filler constitute more than 30% but less than 40% of the weight of the composition. The invention also features use of a composition or device as described herein (*e.g*., a composition that includes, and that may include only, a filler, a copolymer, and an antibiotic) for the preparation of a medicament for the treatment of osteomyelitis.

Similarly, the invention features methods of treating a patient who has bone cancer by administering to the patient (*e.g*., at the site from which a tumor has been excised) a composition comprising a composition or device described herein that includes a chemotherapeutic agent. For example, a patient having a bone cancer can be treated with a composition or device that includes any of the components described herein (*e.g*., poly(lactide-co-glycolide) and calcium carbonate) and a chemotherapeutic agent. As noted in connection with the compositions, the poly(lactide-co-glycolide) can include lactide:glycolide units at a ratio of 85:15, and the calcium carbonate constitute more than 30% but less than 40% of the weight of the composition. The invention thus features use of a composition or device as described herein (*e.g*., a composition that includes and may include only, a filler (e.g., calcium carbonate) and a copolymer (e.g., a copolymer formed from lactic acid monomers and glycolic acid monomers) and a chemotherapeutic agent for the treatment of bone cancer. As noted, the filler can constitute more than 30% but less than 40% of the weight of the composition. The invention also features use of a composition or device as described herein (*e.g*., a composition that includes, and may include only, a filler, a copolymer, and a chemotherapeutic agent) for the preparation of a medicament for the treatment of bone cancer.

As copolymers such as PLGA degrade *in vivo* by hydrolysis into natural metabolic products, the compositions of the present invention and devices or implants made as described herein are biocompatible and may also be referred to as bioabsorbable (*i.e.*, as able to degrade over time in a biological environment such as the human body to compounds that are removed during normal metabolic processes). Moreover, devices fashioned with the present compositions can degrade over a period of time that allows a desirable shift in weight bearing from the device to the patient's own tissues. While the compositions of the invention are not limited to those having any particular advantage, we believe the inclusion of calcium carbonate decreases the rate of acid catalyzed hydrolysis, allowing for greater strength retention suitable for orthopedic repair devices. The release of calcium may stimulate bone cells and accelerate bone repair. The filler may also increase or enhance biocompatibility or dimensional stability, facilitate processing, and/or improve the appearance of the composition.

### BRIEF DESCRIPTION OF THE DRAWINGS

Figure 1 is a table depicting % mass loss on days 1, 2, 4, and 5 from various compositions prepared as described in Example 1.
Figure 2a is a table depicting molecular weight loss for the compositions listed after 1, 2, and 4 days, as described in Example 1.
Figure 2b is a line graph representing the tabular data of Figure 2a.
Figure 3 is a table indicating the pass/fail rating for four compositions (PDLG, PLC15, PLC35, and PLC50) in a standard industry torsional test.
Figure 4 is a line graph depicting the degradation of molecular weight for four compositions (PDLG, PLC15, PLC35, and PLC50) over 26 weeks *in vitro.*
Figure 5 is a line graph depicting the degradation of mass for four compositions (PDLG, PLC15, PLC35, and PLC50) over 52 weeks *in vitro.*
Figure 6 is a bar graph comparing the results of strength retention testing with PLC and PLLA over 24 weeks (as described in Example 3).
Figure 7 is a series of three photographs of an implanted PLC screw at six weeks, 26 weeks, and 52 weeks (left to right) following implantation into the femur of a sheeep.
Figure 8 is a pair of photographs of an implanted PLC screw (left-hand photograph) and a PLLA screw (right-hand photograph) one year following implantion into the femur of a sheep.
Figure 9 is a pair of CT scans. The left-hand scan shows the location of a PLC screw in the femur of a sheep after 52 weeks implantation (the screw was replaced by normal cancellous bone). The right-hand scan shows a PLLA screw after the same period of time. The PLLA screw is still present.
Figure 10 is a pair of photographs of the sites of implantation of a PLC screw six weeks after implantation (left-hand photograph) and 26 weeks after implantation (right-hand photograph).
Figure 11 is a photograph illustrating central placement of a screw completely surrounded by a tendon graft.
Figure 12 is a bar graph comparing the tensile strength (N) in the reconstructed tibial-femoral complex in animals treated with PLC screws and animals treated with PLLA screws.
Figure 13 is a pair of photographs illustrating the ability of a PLC screw to stimulate graft ossification (presumably by calcium release) after 26 weeks implantation (left-hand photograph) and after 52 weeks implantation (right-hand photograph).
Figure 14 is a photograph illustrating a PLLA screw after 52 weeks implantation under the same conditions as the PLC screw shown in Figure 13 (and described in Example 5).
Figure 15 is a series of CT sections through the planes shown as Z1, Z2, and Z3, showing the progression of bone integration in both the graft and PLC screw domains.

### DETAILED DESCRIPTION

As noted, the compositions of the invention can include a co-polymer and a filler material. These components, as well as additional components and methods of use are described further below.

*Copolymers*: As noted, the compositions of the invention can include a copolymer, including copolymers produced from lactide and glycolide monomers. Lacide monomers can be present in the D-form or the L-form. Alternatively, the copolymer can include a combination of monomers in both the D- and L-forms. For example, 20-28% of the lactide monomers (*e.g*., 25-75%, 30-70%, 40-60%, or about 50%) can be D-lactide monomers. As noted, where the co-polymer includes monomers of lactic and glycolic acids, we may refer to it as PLGA, and where both isoforms are present, we may refer to poly(dl-lactide-co-glycolide) (PDLGA). Moreover, the ratio of monomers (*e.g*., the ratio of lactide to glycolide units) can vary. For example, the copolymer can contain 50:50 lactide:glycolide units to 90:10 lactide:glycolide units (*e.g*., 85:15 lactide:glycolide units; as noted above, the ratio can vary from these absolute numbers due to the manufacturing process). The copolymer can be manufactured by methods known to those of ordinary skill in the art or purchased from a commercial supplier.

*Filler material:* Materials suitable for inclusion as fillers with any of the copolymers described herein (*e.g*., with PLGA or PDLGA, for example where the ratio of lactide:glycolide units is 85:15) include basic organic and inorganic metal compounds, such as acetates, lactates, glycolates, hydroxides, carbonates, phosphates, and halides. For example, the filler can be sodium acetate, potassium acetate, sodium lactate, potassium lactate, calcium lactate, potassium glycolate, calcium glycolate, calcium propionate, calcium oxide, calcium hydroxide, calcium carbonate, calcium phosphate family, calcium fluoride, calcium sulphate, magnesium oxide, magnesium hydroxide, magnesium carbonate, magnesium phosphate, sodium phosphate, sodium fluoride, potassium phosphate, potassium fluoride, or combinations thereof. Calcium carbonate is preferred, and may be used as the sole filler or in combination with another filler material.

Like the copolymer, the filler material can be purchased from commercial suppliers or may be synthesized or purified from natural sources. For example, calcium carbonate is found in nature (*e.g*., in natural coral or other marine life). While the filler is preferably pure or substantially pure, it may contain contaminants. For example, the calcium carbonate may be pure or may contain small amounts *(e.g.,* "trace" amounts) of another compound such as MgCO₃, SiO₃, or [FeAl]₂O₃. With respect to form, the calcium carbonate may be particulate, and the particles can be roughly spherical, cubical or tetrahedral measuring in size from very small (*e.g*., less than about 0.10 µm) to quite large (*e.g*., about 10.0 µm or more). For example, the particles can have a diameter of 0.1-0.5 µm; 0.5-2.5 µm; 2.5-5.0 µm; 5.0-7.5 µm; 7.5-10.0 µm; or sizes within the ranges provided (*e.g*., 8.0-9.0 µm). The particles, or a majority of the particles, can be of approximately the same size or they can be of a range of different sizes (*e.g*., the smallest can be about 0.01, 0.05, 0.10, 0.25, 0.50, 0.75,1.0, 1.25, 1.50,1.75, 2.00, or 2.50 µm and the largest can be about 5.0, 6.0, 7.0, 8.0, 9.0, or 10.0 µm). Regardless of size, the particles can be solid or can contain a hollow core, or be porous in nature.

The amount of the filler within the composition can vary. For example, where the composition contains only a copolymer and filler, the filler constitute 20-50% of the composition *i.e.* 30-40% (*e.g*., about 35%) by weight. For example, where the total weight of a composition is 100 g, it can include 60-70 g of a copolymer and 30-40 g of filler (*e.g*., 65 g of PLGA (*e.g*., PLGA at 85:15 lactide:glycolide units) and 35 g CaCO₃). Where one or more additives are included, as described below, the amount of the filler can nevertheless remain the same (*i.e.*, 20-50% (*e.g.,* 30-40% (*e.g*., about 35%))) of the composition as a whole. Alternatively, the filler can constitute 20-50% (*e.g.,* 30-40% (*e.g.*, about 35%)) by weight of the weight of the copolymer.

*Additives:* If desired, any of the compositions described herein (*e.g.,* a mixture of PLGA (*e.g*., 85:15 lactide:glycolide units) and calcium carbonate), regardless of form, can contain one or more additives (*e.g*., therapeutic agents such as biotherapeutics or pharmaceuticals). For example, a calcium carbonate-PLGA composition (*e.g*., Poly Lactide Carbonate (PLC)) fashioned as a tissue fixation device or material for orthopedic application (*e.g*., a bone graft substitute) can include one or more additives (*e.g*., therapeutic agents). The additive(s) can be released as the device degrades or absorbs *in vivo.* Alternatively, or in addition, an additive can diffuse away from an intact device or can be positioned on the surface of the device so that it exerts an effect (*e.g*., an effect on surrounding tissue) after implantation. Accordingly, an additive may be incorporated throughout the device (*e.g*., it may form part of a substantially homogeneous device) or it may be spatially segregated (*e.g*., in an inner compartment or on the device's surface).

The therapeutic agent can be, or can include, a growth factor, including growth factors such as those from the fibroblast growth factor family, transforming growth factor family, or platelet derived growth factor family that act as chemoattractants and/or growth stimulators, a hormone such as human growth hormone, an antibiotic, an antiviral agent, an antifungal agent, an anti-inflammatory agent, an inflammatory mediator such as an interleukin, tumour necrosis factor, a prostaglandin, nitric oxide, an analgesic agent, an osteogenic factor such as a bone morphogenetic protein, or a matrix molecule such as hyaluronan. Other agents include angiogenic factors that are materials capable of directly or indirectly promoting angiogenesis. Examples include angiogenic peptide growth factors in autologous, xenogenic, recombinant, or synthetic forms (*e.g*., a member of the vascular endothelial growth factor family). Further examples are blood clot breakdown products, such as thrombin and heparin including autologous, allogeneic, xenogeneic, recombinant and synthetic forms of these materials. Compositions based around butyric acid, including butyric acid (butanoic acid, C₄H₈O₂) and butyric acid salts, including sodium, potassium, calcium, ammonium and lithium salts, α-monobutyrin (1-glycerol butyrate; 1-(2,3 dihydroxypropyl) butanoate; C₇H₁₄O₄) and hydroxybutyrate can also be incorporated. The therapeutic agent can also be a chemotherapeutic, cytotoxic, or immunotherapeutic agent. For example, the compositions can contain doxorubicin hydrochloride (Adriamycin), methotrexate with citrovorum, cisplatin, vincristine, cyclophosphamide, and/or dacarbazine.

Where antibiotics are incorporated, the compositions of the invention can be used to treat osteomyelitis and may be administered prophylactically (*e.g*., in the event of bone surgery).

These therapeutic agents and other additives can be provided in physiologically acceptable carriers, including within sustained-release or timed-release formulations. Acceptable pharmaceutical carriers are well known in the art and are described, for example, in *Remington's Pharmaceutical Sciences* (Mac Publishing Co., A.R. Gennaro Ed.). Carriers are non-toxic to recipients at the dosages and concentrations employed, and include diluents, solubilizers, lubricants, suspending agents, encapsulating materials, solvents, thickeners, dispersants, buffers such as phosphate, citrate, acetate and other organic acid salts, anti-oxidants such as ascorbic acid, preservatives, low molecular weight peptides (*e.g*., peptides having less than about 10 residues) such as polyarginine, proteins such as serum albumin, gelatin or an immunoglobulin, hydrophilic polymers such as poly(vinylpyrrolindinone), amino acids such as glycine, glutamic acid, aspartic acid or arginine, monosaccharides, disaccharides, and other carbohydrates including cellulose or its derivatives, glucose, mannose or dextrines, chelating agents such as EDTA, sugar alcohols such as mannitol or sorbitol, counter-ions such as sodium, and/or non-ionic surfactants such as tween, pluronics, or polyethyleneglycol (PEG). Moreover, the additives can be linked to agents that facilitate their delivery. For example, an additive can be linked to an antibody or antigen-binding fragment thereof, including a single chain antibody, a growth factor, hormone, or other ligand that specifically binds a target (*e.g*., a cell surface receptor).

The substances within the compositions can be combined in any order. For example, the calcium carbonate and PLGA can be combined before the additive is introduced or all three types of ingredients (the filler, the copolymer, and the additive) can be combined at essentially the same time. The additive may be dissolved in a carrier (including those described above) and combined with a stabilizer or other agent (*e.g*., the targeting agents described above) before it is combined with another component.

The amount of additive incorporated into the composition can vary, but will be a therapeutically effective amount (*i.e.*, an amount that confers a therapeutic benefit on the subject treated with the composition). To help preserve the composition, it can be packaged and stored under conditions in which the activity of the additive is likely to be preserved (*e.g*., ambient or cool temperatures (*e.g*., 4°C)).

Therapeutically effective dosages may be determined by studies conducted *in vitro* or *in vivo.* Determining effective dosage levels (*i.e.*, the dosage required to achieve a desired result) is well within the abilities of one of ordinary skill in the art. The position of the additive within the device and the rate at which it is released can also be varied to determine an optimal or acceptable rate of delivery. A typical additive dosage can range from 0.001 mg/kg to 1000 mg/kg, preferably from 0.01 mg/kg to 100 mg/lkg, and more preferably from 0.10 mg/kg to 20 mg/kg. The additives may be used alone or in combination with one another or with diagnostic agents.

*Manufacturing:* The filler material (*e.g*., calcium carbonate) and, optionally, an additive can be incorporated into the copolymer by any means known in the art (*e.g*., mixing, stirring, shaking, milling, melt blending, or any other blending technique). Once incorporated, the combined materials can be formed into a device (*e.g*., a medical device, implant, or prosthesis, such as those described above). The device can be a tissue fixation device or it can be a material or device suitable for orthopedic application (*e.g*. the compositions of the invention can be used as bone graft substitutes, spinal fusions, bone plates, bone plate screws, and the like). We may refer to bone substitute materials as "synthetic bone substitutes." The device can be fabricated by any method that involves a physical or phase change of the material or its components in order to form a specific resin, geometry, or product. For example, a device can be formed by an extrusion process (*e.g*., a single screw, twin screw, disk, ram, or pulltrusion process); a molding process (*e.g*., an injection, intrusion, compression, or thermoforming process); a solvent based process (*e.g*., mixing or casting); a welding process (*e.g*., an ultrasonic or hermetic process); a polymerization process (*e.g*., reaction injection molding, bulk polymerization, and solvent polymerization); or by other methods (*e.g*., fiber spinning or electrospinning). Pellets, powders, or other physical forms of the copolymer (*e.g*., pellets, granules, or interlocking shapes) can be coated with powders of the filler (*e.g*., calcium carbonate) with blending occurring in an extruder, which may be employed in the subsequent processing of the polymer to provide a useful medical device. Such devices include screws, pins, rods, plates, sutures, suture anchors, staples, clips, rings, and the like. In the case of a suture, the construction can produce a monofilament or multifilament suture (*e.g*., a braided, twisted, or spun suture made by conventional techniques such as those described in U.S. Patent No. 5,019,093).

When intended for use as a synthetic bone substitute or an "infilling" item, the compositions can be fashioned into a paste-like product, which can be readily used to fill bone cavities or irregularities. The compositions described herein can be used as synthetic bone substitutes to treat injuries that result from trauma, surgery, or degenerative conditions that affect bone. Such substitutes provide an alternative to the use of autologous or allogeneic bone, and they can provide a matrix to facilitate bone growth and healing. We mention "infilling" above. The compositions described herein can be used to fill a donor site when an autologous bone graft is taken for use in another anatomical location. More specifically, the compositions described herein can be used in, or fashioned for use in, joint fusions, fracture treatment (*e.g*., fresh and non-union), revision hip procedures, and osteotomies.

In one embodiment, the filler (*e.g*., a CaCO₃ powder) can be added to a solution of the copolymer in an organic solvent, which is subsequently evaporated. Evaporation of the solvent (*e.g*., chloroform) can be facilitated by stirring or otherwise agitating the solution. Any residual solvent can then be removed in a vacuum oven. The sold mixture obtained may then be compression molded at a temperature at least equal to the softening temperature. The molded solid items can, if necessary or desired, be machined to a particular shape (*e.g.*, the shape of a bone fragment they are meant to replace).

Compositions (*e.g*., amorphous compositions) and polymer-based devices used for medical purposes should also be sterile. Sterility may be readily accomplished by conventional methods such as irradiation or treatment with gases or heat, an electronic beam (e beam), or light (*e.g*., white light). For example, the polymer-based compositions described herein can be sterilized through steam sterilization (*e.g*., by autoclaving), treatment with ethylene oxide (EtO) gas, or exposure to radiation (*e.g.,* γ irradiation) (*see, e.g.,* Athanasiou et al., Biomaterials 17:93-102, 1996; Baker et al., J. Biomed. Mat. Res. 46:112-120, 1999; Besong et al., J. Bone Joint Surg. 80-B:340-344, 1998; Buchanan et al., Biomaterials 20:823-837, 1999; Costa et al., Biomaterials 19:659-668, 1998; Dillow et al., Proc. Natl. Acad. Sci. USA 96:10344-10348, 1999; Gogolewski and Mainil-Varlet, Biomaterials 17:523-528, 1996; Gogolewski and Mainil-Varlet, Biomaterials 17:251-255, 1997; Kurtz et al., J. Biomed. Mat. Res. 46:573-581, 1999; Kurtz et al., Biomaterials 20:1659-1688, 1999; Pascaud et al., Biomaterials 18:727-735, 1997; Ratner et al., Eds., Biomaterials Science: An Introduction to Materials in Medicine, Academic Press, pp. 415-420, 1996; and Sauer et al., Biomaterials 17:1929-1935, 1996).

Steam sterilization is a common form of sterilization that sterilizes materials by exposing them to high temperature steam (over about 121°C), under pressure (about, or more than, two atmospheres), for about 15-30 minutes. As autoclaving can harm polymeric biomaterials, an alternate method of sterilization may be preferable. As noted, the compositions described herein can also be sterilized by exposure to EtO gas, which kills microorganisms by alkylating the amine groups on nucleic acids. To prevent or reduce toxicity (EtO can attack the same amine groups in humans that it attacks in microorganisms), materials sterilized with EtO can be washed (*e.g*., washed 2-10 times with air) (Kurtz et al., Biomaterials 20:1659-1688, 1999 and Ratner *et al.,* Eds., *supra*). Radiation (*e.g*., γ radiation) sterilizes materials by ionizing the nuclei acids of any contaminating microorganisms. A typical application is of 60Co at 25 - 40 kGy). If required, more detailed procedures for sterilizing materials by these methods are readily available, and one of ordinary skill in the art is easily able to perform them. Accordingly, the methods of manufacturing a polymer-based composition (*e.g*., a PLGA/CaCO₃-containing composition) can include the step of sterilizing the composition.

Regardless of the precise method by which the compositions are sterilized, the goal is to remove (or destroy or disable) living organisms (*e.g*., bacteria) or other disease-causing agents (*e.g*., viruses, fungi, yeast, molds, and prions) from (or within) the composition. Sterility is generally quantified using the sterility assurance limit (SAL) and process conditions determined by performing fractional sterilization runs. The SAL is the probability that a given implant will remain nonsterile following a sterilization run, and the accepted minimum value for the SAL is 10-6. At that value, one implant in one million may be nonsterile.

*Use:* The compositions and devices can be used in a wide variety of situations to treat patients who have experienced an injury (exemplary tissue fixation devices and materials for orthopedic application are described above). While human patients are clearly candidates for treatment, the invention is not so limited. Veterinary application is also possible (the animals may be domesticated pets (such as dogs or cats), farm animals (*e.g*., horses, cows, goats, pigs, or sheep), laboratory animals (such as rodents or non-human primates), or wild animals (*e.g*., a non-human primate or other mammal (*e.g*., an animal kept in a zoo)). The compositions can also be used in the event of elective surgery, including cosmetic surgery. The method may be one in which soft tissue is attached to bone or one in which the primary site of repair is bone *per se.* The process can encompass any type of tissue modification, including tissue repair, reconstruction, remodeling, and tissue-guided regeneration, including wholly internal processes as well as processes that include or affect the skin or an orifice such as the mouth or nose (*e.g*., the compositions of the invention can be used in dental procedures).

### EXAMPLES

### Example 1: Poly-dl-lactide-co-glycolide (PDLG) (85:15) with CaCO₃ or CaSO₄

The studies described here were designed to evaluate the hypothesis that basic fillers such as calcium carbonate and calcium sulfate delay the degradation rate of amorphous polymers, including PDLG having about 85% lactide units and 15% glycolide units. We used dried PDLG 85:15 with an initial intrinsic viscosity (I.V.) of 1.16. The calcium carbonate and calcium sulfate had a purity of over 99%.

To mix the copolymer and filler, we began by dissolving various components in a solvent. Each of the following were dissolved in 150 ml chloroform: (1) 15 g of PDLG; (2) 9 g of PDLG and 6 g of calcium carbonate; and (3) 9 g of PDLG and 6 g of calcium sulphate. The materials were allowed to dissolve in the solvent over several hours. The solutions were agitated periodically and then emptied out onto a glass tray. As the solvent evaporated, a thin film of mixed polymer and filler formed on the tray. The film was peeled off the tray and compression molded as described below. PDLG resin was also compression molded directly.

To compression mold the materials, we preheated a compression molder to 150°C. We placed the mold onto the lower mold plate, and filled the cavity with approximately 15 grams of material before inserting it into the compression molder. The material sat for approximately five minutes or until the polymer resin began to adhere to itself. We then increased the heat to approximately 180°C and let the material sit until a consistent melt had formed. The top mold plate was placed onto the bottom mold plate, and the mold clamp was screwed down to compress the sample. After 10 seconds, we released the pressure to allow gasses escape, then reapplied the pressure and let sample cure for 30-60 seconds. The mold was removed, quenched under cold water, and opened using a rubber mallet. We used a band saw to cut the disc into parts (0.5" x 0.75"), which were placed in 100 mls of a buffer solution at 67°C. Samples were removed from the solution at time zero and after 1, 2, 4, 7, or 9 days, and dried to constant weight at 50°C under vacuum. Mass loss was recorded before the samples were subjected to GPC analysis. Their thickness was also measured before and after degradation.

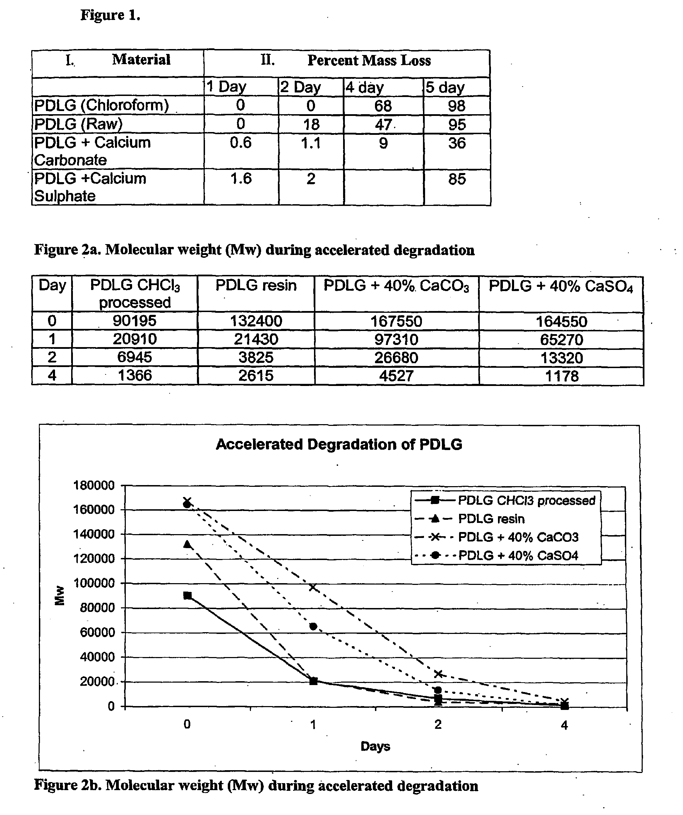

The percentage mass loss is shown through day five in the table of Figure 1, and the loss of molecular weight is presented in tabular and graphical form in Figures 2a and 2b, respectively. The results clearly demonstrate that the degradation of poly dl lactide co-glycolide is retarded by the addition of calcium sulphate and calcium carbonate. This can be seen in both the molecular weight loss and the mass loss of the polymer. Calcium carbonate was more effective in slowing the degradation rate than calcium sulphate.

### Example 2 Degradation Studies of Molded Implants

The purpose of this study was to evaluate poly(dl-lactide-co-glycolide (85:15)) (PDLG) blended with calcium carbonate, as a material for bioabsorbable medical devices, specifically interference screws. We evaluated *in vitro* degradation characteristics to determine the effect of calcium carbonate on the rate of degradation of these polymers in a molded form and assessed the materials for initial torsional strength.

The pure polymer was molded following drying using a standard molding procedure into an interference fixation screw. We produced filled material by blending calcium carbonate into PDLG. The weight of the filler, as a percentage of the polymer, was 15, 35, or 50%. The resulting material is designated poly lactide carbonate (PLC); materials containing 15% calcium carbonate are designated PLC15; those containing 35% are designated PCL35; and those containing 50% are designated PLC50. The materials were molded according to standard molding procedures into an interference fixation screw and tested for torsional strength. A pass/fail criteria based on industry specifications was used to determine if the materials had sufficient torsional strength to be used as interference screws.

For *in vitro* degradation testing, each screw was placed in phosphate buffered saline (PBS) and maintained at a temperature of 37°C. The incubated samples were assessed for molecular weight, and mass loss at 0, 2, 4, 6, 8, 10,12, 26, and 52 weeks. The molecular weight of the degraded samples was analyzed using chloroform GPC and compared with the starting material to evaluate degradation during *in vitro* conditioning.

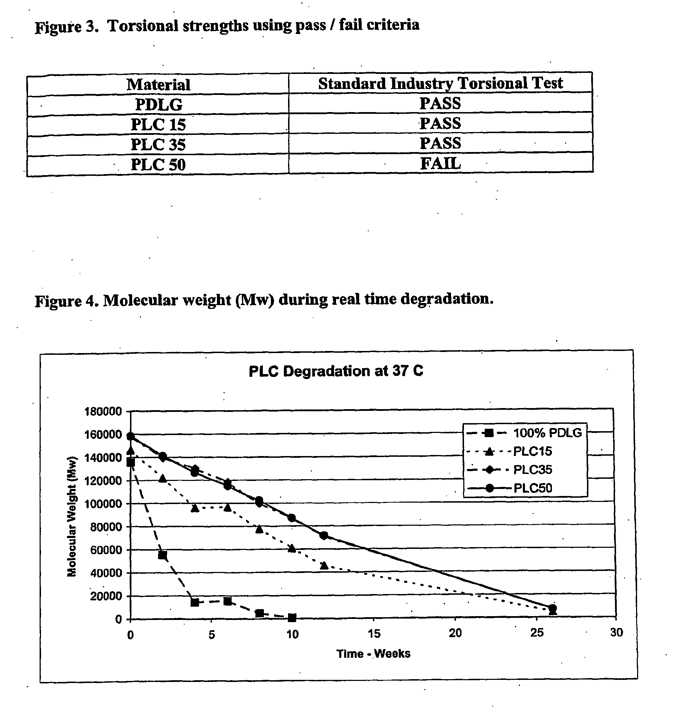

The torsional test results shown in Figure 3 indicate that that PDLG and PLC15 and PLC35 have acceptable torsional strength. PLC50 failed this test indicating that the filler level is too high and this material is not well suited for biomedical screw applications.

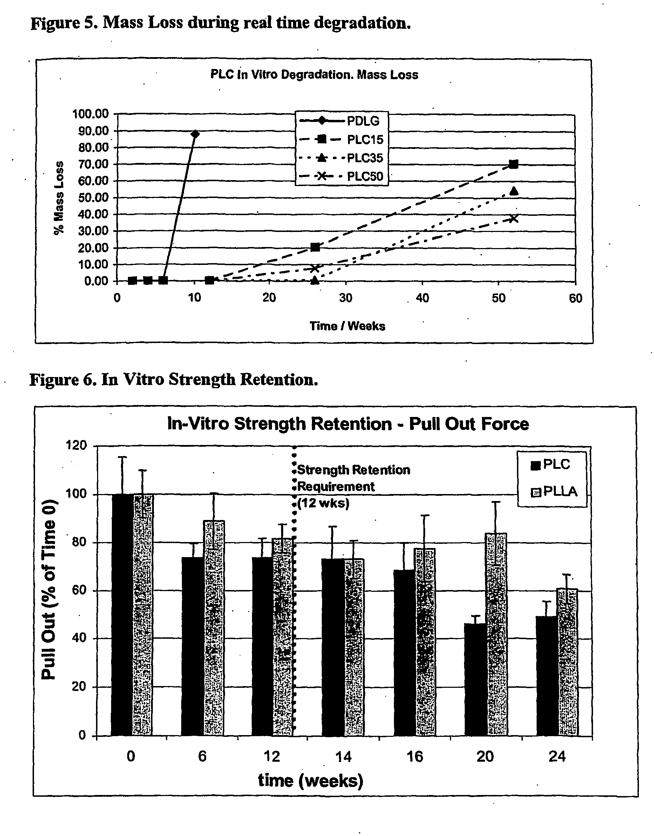

The loss in molecular weights, depicted in Figure 4, clearly shows the effect of calcium carbonate on the degradation rate. The rate is slowed down by addition of calcium carbonate. This is proportional to the mass ratio of the calcium carbonate in the PLC until 35% is reached. No difference could be seen between PLC35 (35% calcium carbonate) and PLC50 (50% calcium carbonate). Mass loss data (shown in Figure 5) also clearly demonstrates the effect of calcium carbonate on PDLG. Samples of PDLG showed considerable mass loss (88%) after 10 weeks *in vitro.* For samples of PLC15, mass loss began between 12 and 26 weeks *in vitro,* as 20% mass loss was realized at 26 weeks. No significant mass loss was shown at 26 weeks for samples of PLC35, and PLC50. Samples of all three PLC blends showed significant mass loss at 52 weeks (70%, 54%, and 30%, for PLC15, PLC35, and PLC50, respectively). An ASH test was performed on the degraded materials and, assuming no mass loss was attributed to calcium carbonate, the materials had all lost nearly 90% of their polymer portion.
Our conclusions from this study are as follows: (1) the degradation rate of poly(dl-lactide-co-glycolide) is too fast for fixation device applications that require strength retention to 12 weeks; (2) the addition of calcium carbonate decreases the rate of degradation in proportion to the amount of calcium carbonate in the polymer until around 35-40% by weight; (3) Initial torsion testing indicated torsion strength for this design device is below acceptable levels for the composition with 50% calcium carbonate. Based on these studies, we considered further analysis of PLC with about 35% calcium carbonate. This formulation contained enough calcium carbonate to slow the degradation rate and thereby enhance strength retention, but not so much calcium carbonate that the initial mechanical properties of the compositions were compromised.

### Example 3: Further degradation studies (strength retention)

This study was designed to evaluate the *in vitro* mechanical characteristics of a sterilized tibial fixation screw (7x9x25 mm) produced from Poly Lactide Carbonate (PLC); poly-dl lactide-coglycolide (85:15) blended with calcium carbonate 65:35 w/w. We evaluated the material for strength retention characteristics and used poly-1-lactide (PLLA) tibial fixation screws as controls.

To test strength retention, we cut saw bone (20 pcf) into cubes (4x4x4 cm) and drilled an 11 mm hole through the center of each cube. We then cut leather straps (25.5 x 1.5 cm) from standard 1.5 mm thick leather (natural vegetable KIP, grade A), folded it in half, and inserted it through the hole to form a loop coming out the other side of the cube. We took care to position the leather within the hole to ensure the strap followed the circumference of the hole, forming a channel in the center of the strap. The screw was then inserted down this channel until the head of the screw was just below the surface of the saw bone.

We placed each saw bone block containing a screw and leather strap into a 500 ml sealed jar filled with PBS, and placed the jar in a water bath at 37°C. Samples were removed one day, 6, 12,14, 16, 20, 24 weeks later for mechanical testing. For both the experimental (PLC) screw and the control (PLLA) screw, ten replicates were performed at each time point.

The samples were tested to failure by placing the bone block under a standard Instron base grip. The loop of the leather was attached to a hook fixed to the load cell of the Instron and pulled to failure at 1 mm/second. The results are shown in Figure 6. No significant difference (p=0.01) was seen between the two materials at any of the three time points to 12 weeks. Therefore, mechanical pull-out testing has shown that screws made from PLC retain fixation strength comparable to that of screws made from PLLA for at least 12 weeks.

### Example 4: Evaluation of a Tapered Screw in an Ovine Model

PLC and PLLA screws were implanted directly into the cancellous bone of the left medial distal femur of an ovine model. Histology and computed tomography (CT) were performed over time to assess biocompatibility and bone integration into the screws.

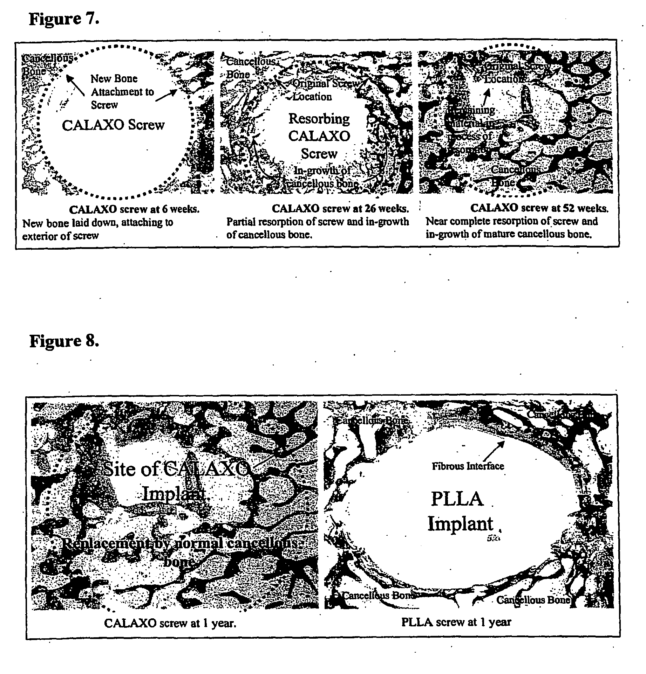

The histological analysis performed on the PLC screw revealed new bone formation at all time points examined, starting with new bone formation and attachment around the margin of the screw at six and 12 weeks (Figure 7, left-hand photograph). At 26 weeks the PLC screw was partially integrated with bone (Figure 7, center photograph), and at 52 weeks, the screw was replaced with new bone (Figure 7, right-hand photograph). In contrast, the PLLA screw was still present and surrounded by fibrous tissue even after 52 weeks implantation. These results indicate that the PLC screw is osteoconductive. The amount of bone formation increased with time in the group of animals that received the PLC screw, as the screw degraded and was replaced by cancellous bone. At one year, the implant site was fully healed with normal cancellous bone. Even after this extended period of time, the PLLA screw was fully present; there was no sign of resorption (Figure 8). These results are consistent with our prior studies demonstrating that PLLA degrades extremely slowly and is not replaced by bone.

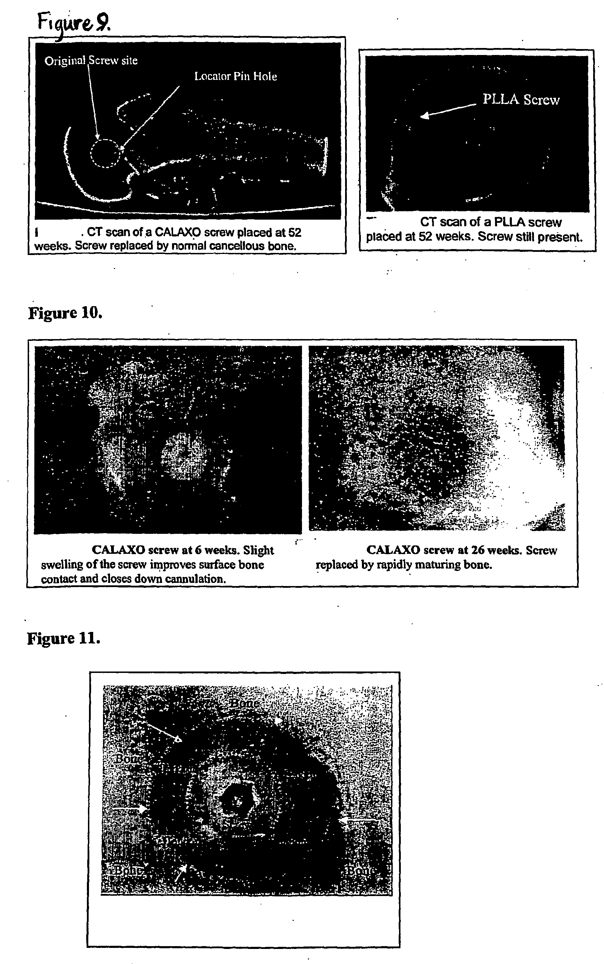

Computed tomography results for the PLC screw showed extensive bone integration at 26 weeks and new bone formation by 52 weeks. The new bone formation was so extensive that no evidence of the screw could be seen. These results support our belief that the PLC screw is osteoconductive. The PLLA screw was still present at 52 weeks in all animals tested (*see* Figure 9).

Macroscopically, the PLC screws were easily seen at 6 and 12 weeks following implantation. It was difficult to identify the PLC screw after 26 weeks, and it was not possible after 52 weeks due to the extent of bone integration. We believe the slight swelling of the PLC screw improves surface-bone contact and closes down cannulation *(see* Figure 10, left-hand photograph). After 26 weeks, the PLC screw was in the process of being replaced by rapidly maturing bone (*see* Figure 10, right-hand photograph).

Based on this study, we concluded that: (1) when placed directly in cancellous bone, the PLC screw was gradually replaced with normal bone and is, therefore, osteoconductive; (2) PLLA screws remain present in cancellous bone for at least 52 weeks; (3) the PLC material is biocompatible (bone attachment was seen at the earliest time point studied); and (4) the combination of an amorphous bioabsorbable polymer and calcium carbonate is ideal for use in devices such as sports medicine fixation devices.

### Example 5: In vivo A CL study

A PLC screw was compared to a PLLA interference screw in a soft tissue ACL model. The screws were placed in the center of a four-stranded graft, which represents the worst-case scenario for bone integration, as the screw is fully encapsulated with tendon tissue *(see* Figure 11). This model is unlike many fixation techniques, where the screw is placed alongside the graft and in contace with bone that will enhance bone integration.

Mechanical testing was performed to assess overall repair strength and filure modes to 12 weeks (n=10). This time point was chosen because it is well established that graft/tunnel healing and fixation occurs in approximately four weeks using bone-tendon-bone (BTB) grafts and before 12 weeks using soft tissue grafts in ACL repair (Grana et al., Am. J. Sports Med. 22:344-351, 1994; Rodeo et al., J. Bone Joint Surg. 75-A:1795-1803, 1993; Weiler et al., Arthroscopy 18:113-123, 2002).

Histology and CT were used to assess biocompatibility, tendon-bone integration and bone formation. These tests were performed at 6, 12, 26, and 52 weeks following implantation, with six replicates at each time point.

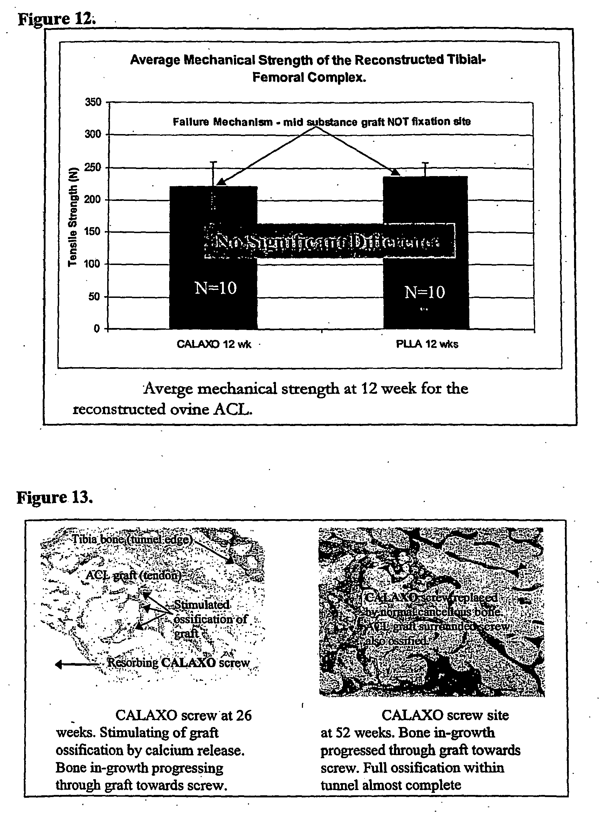

We did not observe any difference in mechanical properties of the repaired ACL in animals treated with the PLC screw and animals treated with the PLLA interference screws (the results obtained at 12 weeks are shown in the graph of Figure 12).

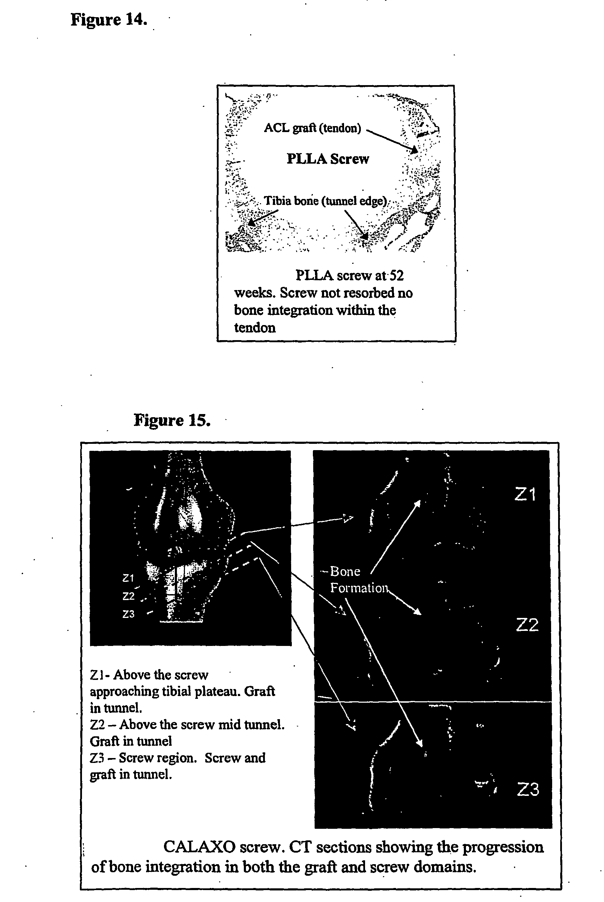

Our histological analysis demonstrated that, within one year, the PLC screw was replaced by bone, and the material also stimulated bone formation in the tendon graft within the tunnel. Figure 13 illustrates the stimulating effect the PLC screw has on the surrounding graft tissue. Ossification of the graft can clearly be seen in the tunnel containing the PLC screw, but no ossification was seen around the PLLA screw. Ossification was stimulated in the PLC-repaired graft by 26 weeks and both the PLC screw and the surrounding ACL graft were ossified by 52 weeks (Figure 13). New bone formation was noted within the tendon graft in only the PLC-treated group. The PLLA screw remained intact and inert at 52 weeks (Figure 14).

The PLC screw also stimulated the ossification of the tendon graft away from the screw position but still within the tunnel. This ossification was not seen in animals treated with the PLLA screw (Figure 14). Thus, our histological analyses support the hypotheses that the PLC screw is replaced by bone when placed in an osseous site; is an osteoconductive material; and actively stimulates ossification of the tendon graft within the bone tunnel.

CT was performed to examine bone formation with the bone tunnel for both the PCL and PLLA screws. The PLLA screws were present at all time points examined with no demonstrable *in vivo* resorption. The PLC screws were replaced by bone and bone formation was noted throughout the tunnel within the graft indicating the bone-stimulating effect of PLC. CT sections in three planes, showing the progression of bone integration in both the graft and screw domains are shown in Figure 15.

These studies support the following conclusions: (1) PLC screws are biocompatible and exhibit fixation strength equivalent to the PLLA screws (both providing adequate mechanical fixation until healing had occurred); (2) the PLC material was osteoconductive, facilitating in-growth of bone into the implant material; and (3) the PLC screws actively stimulated bone formation with a tendon graft that was present in the bone tunnel. Further, the inta-articular portion of the graft, articular cartilage and synovium was normal throughout the study for both PLC-treated and PLLA-treated animals. Thus, the PLC screws are useful as a healing material and may be ideal for use in interference screws used for ACL reconstruction.

## Claims

1. A biocompatible composition comprising calcium carbonate and a copolymer formed from lactic acid monomers and glycolic acid monomers, **characterized in that** the calcium carbonate constitutes more than 30% but less than 40% of the weight of the composition.

2. The biocompatible composition of claim 1, wherein the calcium carbonate has the crystalline structure of calcite.

3. The biocompatible composition of claim 1 or claim 2, wherein the calcium carbonate is present as calcium carbonate particles of a substantially uniform size.

4. The biocompatible composition of claim 3, wherein a majority of the calcium carbonate particles are 0.1-0.5 µm in size; 0.5-2.5 µm in size; 2.5-5.0 µm in size; 5.0-7.5 µm in size; or 7.5-10.0 µm in size.

5. The biocompatible composition of claim 1 or claim 2, wherein the calcium carbonate is present as calcium carbonate particles ranging in size from 0.01 µm to 10.0 µm.

6. The biocompatible composition of any of claims 1-5, wherein the lactic acid monomers are L-form lactic acid monomers.

7. The biocompatible composition of any of claims 1-5, wherein the lactic acid monomers are D-form lactic acid monomers.

8. The biocompatible composition of any of claims 1-5, wherein the lactic acid monomers are a mixture of L-form and D-form lactic acid monomers.

9. The biocompatible composition of any of claims 1-8, wherein the copolymer is poly(dl-lactide-co-glycolide).

10. The biocompatible composition of any of claims 1-9, wherein the ratio of lactic acid monomers:glycolic acid monomers is 85:15.

11. The biocompatible composition of any of claims 1-10, wherein the copolymer is an amorphous copolymer.

12. The biocompatible composition of any of claims 1-11, wherein the calcium carbonate and the copolymer form a substantially homogeneous mixture.

13. The biocompatible composition of any of claims 1-12, wherein the composition is formulated as a powder, a paste, pellets, granules, or interlocking shapes.

14. The biocompatible composition of any of claims 1-13, wherein the calcium carbonate constitutes at least 30% to about 34% of the composition.

15. The biocompatible composition of any of claims 1-13, wherein the calcium carbonate constitutes 34% to 36% of the composition

16. The biocompatible composition of claim 15, wherein the calcium carbonate constitutes about 35% of the composition.

17. The biocompatible composition of any of claims 1-13, wherein the calcium carbonate constitutes about 36% to less than 40% of the composition.

18. The biocompatible composition of any of claims 1-17, further comprising a therapeutic agent.

19. The biocompatible composition of any of claims 1-18, wherein the composition is sterile.

20. A method of making an internal fixation device, the method comprising
(a) providing calcium carbonate;
(b) providing a copolymer formed from lactic acid monomers and glycolic acid monomers;
(c) combining the calcium carbonate with the copolymer to produce a composition, wherein the amount of the calcium carbonate constitutes more than 30% and less than 40% of the composition; and
(d) molding the composition to produce an internal fixation device.

21. The method of claim 20, further comprising step (e): sterilizing the internal fixation device to produce a sterilized fixation device.

22. The method of claim 20 or claim 21, wherein the combining is achieved by an extrusion method.

23. The method of claim 21, wherein sterilizing the internal fixation device is carried out by exposing the device to radiation.

24. The method of any of claims 20-23, wherein the internal fixation device or the sterilized internal fixation device is a screw, pin, rod, plate, suture, suture anchor, staple, clip, or ring.

25. The method of any of claims 20-24, wherein the calcium carbonate has the crystalline structure of calcite.

26. The method of any of claims 20-25, wherein the calcium carbonate is in the form of particles of a substantially uniform size.

27. The method of claim 26, wherein a majority of the calcium carbonate particles are 0.1-0.5 µm in size; 0.5-2.5 µm in size; 2.5-5.0 µm in size; 5.0-7.5 µm in size; or 7.5-10.0 µm in size.

28. The method of any of claims 20-25, wherein the calcium carbonate is present as calcium carbonate particles ranging in size from 0.01 µm to 10.0 µm.

29. The method of any of claims 20-28, wherein the lactic acid monomers are L-form lactic acid monomers or D-form lactic acid monomers.

30. The method of any of claims 24-28, wherein the lactic acid monomers are a mixture of L-form and D-form lactic acid monomers.

31. The method of any of claims 20-30, wherein combining the calcium carbonate and the copolymer comprises forming a substantially homogeneous mixture.

32. The method of any of claims 20-31, wherein the calcium carbonate constitutes 34% to 36% of the composition.

33. The method of claim 32, wherein the calcium carbonate constitutes about 35% of the composition.

34. The method of any of claims 20-31, wherein the calcium carbonate constitutes 36% to 40% of the composition.

35. The method of any of claims 20-34, further comprising providing a therapeutic agent and combining the therapeutic agent with the copolymer and the calcium carbonate.

36. An internal fixation device made by the method of any of claims 20-35.

37. A kit comprising the internal fixation device of claim 36.

38. An internal fixation device comprising the biocompatible composition of any of claims 1-19.

39. The internal fixation device of claim 38, wherein the device is a screw, pin, plate, nail, or suture anchor.

40. Use of a composition or device according to any of claims 1-19 for the manufacture of a medicament for the repair or remodeling of tissue.

41. Use of a composition or device according to any of claims 1-19 for the manufacture of a medicament for the treatment of osteomyelitis.

42. Use of the composition or device according to claim 41, wherein the composition or device comprises an antibiotic, calcium carbonate and a copolymer formed from lactic acid monomers and glycolic acid monomers, wherein the calcium carbonate constitutes more than 30% but less than 40% of the weight of the composition.

43. Use of a composition or device according to any of claims 1-19 for the manufacture of a medicament for the treatment of bone cancer.

44. Use of the composition or device according to claim 43, wherein the composition or device comprises a chemotherapeutic agent, calcium carbonate and a copolymer formed from lactic acid monomers and glycolic acid monomers, wherein the calcium carbonate constitutes more than 30% but less than 40% of the weight of the composition.

## Patentansprüche

1. Eine biokompatible Zusammensetzung, die Calciumcarbonat und ein Copolymer, das aus Milchsäuremonomeren und Glykolsäuremonomeren gebildet ist, beinhaltet, **dadurch gekennzeichnet, dass** das Calciumcarbonat mehr als 30 % aber weniger als 40 % des Gewichts der Zusammensetzung ausmacht.

2. Biokompatible Zusammensetzung gemäß Anspruch 1, wobei das Calciumcarbonat die Kristallstruktur von Calcit aufweist.

3. Biokompatible Zusammensetzung gemäß Anspruch 1 oder Anspruch 2, wobei das Calciumcarbonat als Calciumcarbonatpartikel in einer im Wesentlichen einheitlichen Größe vorliegt.

4. Biokompatible Zusammensetzung gemäß Anspruch 3, wobei ein Großteil der Calciumcarbonatpartikel 0,1-0,5 µm groß ist; 0,5-2,5 µm groß ist; 2,5-5,0 µm groß ist; 5,0-7,5 µm groß ist; oder 7,5-10,0 µm groß ist.

5. Biokompatible Zusammensetzung gemäß Anspruch 1 oder Anspruch 2, wobei das Calciumcarbonat als Calciumcarbonatpartikel in einer Größe im Bereich von 0,01 µm bis 10,0 µm vorliegt.

6. Biokompatible Zusammensetzung gemäß einem der Ansprüche 1-5, wobei die Milchsäuremonomere Milchsäuremonomere in L-Form sind.

7. Biokompatible Zusammensetzung gemäß einem der Ansprüche 1-5, wobei die Milchsäuremonomere Milchsäuremonomere in D-Form sind.

8. Biokompatible Zusammensetzung gemäß einem der Ansprüche 1-5, wobei die Milchsäuremonomere eine Mischung aus Milchsäuremonomeren in L-Form und D-Form sind.

9. Biokompatible Zusammensetzung gemäß einem der Ansprüche 1-8, wobei das Copolymer Poly(D,L-lactid-co-glycolid) ist.

10. Biokompatible Zusammensetzung gemäß einem der Ansprüche 1-9, wobei das Verhältnis von Milchsäuremonomeren zu Glykolsäuremonomeren 85:15 ist.

11. Biokompatible Zusammensetzung gemäß einem der Ansprüche 1-10, wobei das Copolymer ein amorphes Copolymer ist.

12. Biokompatible Zusammensetzung gemäß einem der Ansprüche 1-11, wobei das Calciumcarbonat und das Copolymer eine im Wesentlichen homogene Mischung bilden.

13. Biokompatible Zusammensetzung gemäß einem der Ansprüche 1-12, wobei die Zusammensetzung als ein Pulver, eine Paste, Pellets, Körnchen oder zusammengebackene Gebilde formuliert ist.

14. Biokompatible Zusammensetzung gemäß einem der Ansprüche 1-13, wobei das Calciumcarbonat mindestens 30 % bis etwa 34 % der Zusammensetzung ausmacht.

15. Biokompatible Zusammensetzung gemäß einem der Ansprüche 1-13, wobei das Calciumcarbonat 34 % bis 36 % der Zusammensetzung ausmacht.

16. Biokompatible Zusammensetzung gemäß Anspruch 15, wobei das Calciumcarbonat etwa 35 % der Zusammensetzung ausmacht.

17. Biokompatible Zusammensetzung gemäß einem der Ansprüche 1-13, wobei das Calciumcarbonat etwa 36 % bis weniger als 40 % der Zusammensetzung ausmacht.

18. Biokompatible Zusammensetzung gemäß einem der Ansprüche 1-17, die ferner ein Therapeutikum beinhaltet.

19. Biokompatible Zusammensetzung gemäß einem der Ansprüche 1-18, wobei die Zusammensetzung steril ist.

20. Ein Verfahren zur Fertigung einer Vorrichtung zur internen Fixierung, wobei das Verfahren Folgendes beinhaltet:
(a) Bereitstellen von Calciumcarbonat;
(b) Bereitstellen eines Copolymers, das aus Milchsäuremonomeren und Glykolsäuremonomeren gebildet ist;
(c) Kombinieren des Calciumcarbonats mit dem Copolymer, um eine Zusammensetzung herzustellen, wobei die Menge an Calciumcarbonat mehr als 30 % und weniger als 40 % der Zusammensetzung ausmacht; und
(d) Formen der Zusammensetzung, um eine Vorrichtung zur internen Fixierung herzustellen.

21. Verfahren gemäß Anspruch 20, das ferner Schritt (e) beinhaltet: Sterilisieren der Vorrichtung zur internen Fixierung, um eine sterilisierte Fixierungsvorrichtung herzustellen.

22. Verfahren gemäß Anspruch 20 oder Anspruch 21, wobei das Kombinieren mittels eines Extrudierverfahrens erzielt wird.

23. Verfahren gemäß Anspruch 21, wobei das Sterilisieren der Vorrichtung zur internen Fixierung durchgeführt wird, indem die Vorrichtung Strahlung ausgesetzt wird.

24. Verfahren gemäß einem der Ansprüche 20-23, wobei die Vorrichtung zur internen Fixierung oder die sterilisierte Vorrichtung zur internen Fixierung eine Schraube, ein Spickdraht, eine Stange, eine Platte, eine Naht, ein Nahtanker, eine Klammer, ein Klipp oder ein Ring ist.

25. Verfahren gemäß einem der Ansprüche 20-24, wobei das Calciumcarbonat die Kristallstruktur von Calcit aufweist.

26. Verfahren gemäß einem der Ansprüche 20-25, wobei das Calciumcarbonat die Form von Partikeln in einer im Wesentlichen einheitlichen Größe hat.

27. Verfahren gemäß Anspruch 26, wobei ein Großteil der Calciumcarbonatpartikel 0,1-0,5 µm groß ist; 0,5-2,5 µm groß ist; 2,5-5,0 µm groß ist; 5,0-7,5 µm groß ist; oder 7,5-10,0 µm groß ist.

28. Verfahren gemäß einem der Ansprüche 20-25, wobei das Calciumcarbonat als Calciumcarbonatpartikel in einer Größe im Bereich von 0,01 µm bis 10,0 µm vorliegt.

29. Verfahren gemäß einem der Ansprüche 20-28, wobei die Milchsäuremonomere Milchsäuremonomere in L-Form oder Milchsäuremonomere in D-Form sind.

30. Verfahren gemäß einem der Ansprüche 20-28, wobei die Milchsäuremonomere eine Mischung aus Milchsäuremonomeren in L-Form und D-Form sind.

31. Verfahren gemäß einem der Ansprüche 20-30, wobei das Kombinieren des Calciumcarbonats und des Copolymers das Bilden einer im Wesentlichen homogenen Mischung beinhaltet.

32. Verfahren gemäß einem der Ansprüche 20-31, wobei das Calciumcarbonat 34 % bis 36 % der Zusammensetzung ausmacht.

33. Verfahren gemäß Anspruch 32, wobei das Calciumcarbonat etwa 35 % der Zusammensetzung ausmacht.

34. Verfahren gemäß einem der Ansprüche 20-31, wobei das Calciumcarbonat 36 % bis 40 % der Zusammensetzung ausmacht.

35. Verfahren gemäß einem der Ansprüche 20-34, das ferner das Bereitstellen eines Therapeutikums und das Kombinieren des Therapeutikums mit dem Copolymer und dem Calciumcarbonat beinhaltet.

36. Eine Vorrichtung zur internen Fixierung, die nach dem Verfahren gemäß einem der Ansprüche 20-35 gefertigt wird.

37. Ein Bausatz, der die Vorrichtung zur internen Fixierung gemäß Anspruch 36 beinhaltet.

38. Vorrichtung zur internen Fixierung, die die biokompatible Zusammensetzung gemäß einem der Ansprüche 1-19 beinhaltet.

39. Vorrichtung zur internen Fixierung gemäß Anspruch 38, wobei die Vorrichtung eine Schraube, ein Spickdraht, eine Platte, ein Nagel oder ein Nahtanker ist.

40. Eine Verwendung einer Zusammensetzung oder einer Vorrichtung gemäß einem der Ansprüche 1-19 zur Erzeugung eines Arzneimittels zur Reparatur oder Ummodellierung von Gewebe.

41. Verwendung einer Zusammensetzung oder einer Vorrichtung gemäß einem der Ansprüche 1-19 zur Erzeugung eines Arzneimittels für die Behandlung von Osteomyelitis.

42. Verwendung der Zusammensetzung oder der Vorrichtung gemäß Anspruch 41, wobei die Zusammensetzung oder die Vorrichtung ein Antibiotikum, Calciumcarbonat und ein Copolymer, das aus Milchsäuremonomeren und Glykolsäuremonomeren gebildet ist, beinhaltet, wobei das Calciumcarbonat mehr als 30 % aber weniger als 40 % des Gewichts der Zusammensetzung ausmacht.

43. Verwendung einer Zusammensetzung oder einer Vorrichtung gemäß einem der Ansprüche 1-19 zur Erzeugung eines Medikaments für die Behandlung von Knochenkrebs.

44. Verwendung der Zusammensetzung oder der Vorrichtung gemäß Anspruch 43, wobei die Zusammensetzung oder die Vorrichtung ein Chemotherapeutikum, Calciumcarbonat und ein Copolymer, das aus Milchsäuremonomeren und Glykolsäuremonomeren gebildet ist, beinhaltet, wobei das Calciumcarbonat mehr als 30 % aber weniger als 40 % des Gewichts der Zusammensetzung ausmacht.

## Revendications

1. Une composition biocompatible comprenant du carbonate de calcium et un copolymère formé à partir de monomères d'acide lactique et de monomères d'acide glycolique, **caractérisée en ce que** le carbonate de calcium constitue plus de 30 % mais moins de 40 % du poids de la composition.

2. La composition biocompatible de la revendication 1, dans laquelle le carbonate de calcium a la structure cristalline de la calcite.

3. La composition biocompatible de la revendication 1 ou de la revendication 2, dans laquelle le carbonate de calcium est présent sous forme de particules de carbonate de calcium de taille substantiellement uniforme.

4. La composition biocompatible de la revendication 3, dans laquelle une majorité des particules de carbonate de calcium ont une taille comprise entre 0,1 et 0,5 µm ; une taille comprise entre 0,5 et 2,5 µm ; une taille comprise entre 2,5 et 5,0 µm ; une taille comprise entre 5,0 et 7,5 µm ; ou une taille comprise entre 7,5 et 10,0 µm.

5. La composition biocompatible de la revendication 1 ou de la revendication 2, dans laquelle le carbonate de calcium est présent sous forme de particules de carbonate de calcium dont la taille est comprise dans la gamme allant de 0,01 µm à 10,0 µm.

6. La composition biocompatible de n'importe lesquelles des revendications 1 à 5, dans laquelle les monomères d'acide lactique sont des monomères d'acide lactique en forme de L.

7. La composition biocompatible de n'importe lesquelles des revendications 1 à 5, dans laquelle les monomères d'acide lactique sont des monomères d'acide lactique en forme de D.

8. La composition biocompatible de n'importe lesquelles des revendications 1 à 5, dans laquelle les monomères d'acide lactique sont un mélange de monomères d'acide lactique en forme de L et en forme de D.

9. La composition biocompatible de n'importe lesquelles des revendications 1 à 8, dans laquelle le copolymère est du poly(dl-lactide-co-glycolide).

10. La composition biocompatible de n'importe lesquelles des revendications 1 à 9, dans laquelle le rapport monomères d'acide lactique/monomères d'acide glycolique est 85/15.

11. La composition biocompatible de n'importe lesquelles des revendications 1 à 10, dans laquelle le copolymère est un copolymère amorphe.

12. La composition biocompatible de n'importe lesquelles des revendications 1 à 11, dans laquelle le carbonate de calcium et le copolymère forment un mélange substantiellement homogène.

13. La composition biocompatible de n'importe lesquelles des revendications 1 à 12, dans laquelle la composition est formulée sous forme de poudre, de pâte, de pastilles, de granules, ou de configurations s'enchevêtrant.

14. La composition biocompatible de n'importe lesquelles des revendications 1 à 13, dans laquelle le carbonate de calcium constitue au moins de 30 % à 34 % environ de la composition.

15. La composition biocompatible de n'importe lesquelles des revendications 1 à 13, dans laquelle le carbonate de calcium constitue de 34 % à 36 % de la composition.

16. La composition biocompatible de la revendication 15, dans laquelle le carbonate de calcium constitue 35 % environ de la composition.

17. La composition biocompatible de n'importe lesquelles des revendications 1 à 13, dans laquelle le carbonate de calcium constitue de 36 % environ à moins de 40 % de la composition.

18. La composition biocompatible de n'importe lesquelles des revendications 1 à 17, comprenant de plus un agent thérapeutique.

19. La composition biocompatible de n'importe lesquelles des revendications 1 à 18, dans laquelle la composition est stérile.

20. Une méthode de réalisation d'un dispositif de fixation interne, la méthode comprenant
(a) fournir du carbonate de calcium ;
(b) fournir un copolymère formé à partir de monomères d'acide lactique et de monomères d'acide glycolique ;
(c) combiner le carbonate de calcium avec le copolymère afin de produire une composition, dans laquelle la quantité de carbonate de calcium constitue plus de 30 % et moins de 40 % de la composition ; et
(d) mouler la composition afin de produire un dispositif de fixation interne.

21. La méthode de la revendication 20, comprenant de plus l'étape (e) : stériliser le dispositif de fixation interne afin de produire un dispositif de fixation stérilisé.

22. La méthode de la revendication 20 ou de la revendication 21, dans laquelle le fait de combiner est obtenu par une méthode d'extrusion.

23. La méthode de la revendication 21, dans laquelle le fait de stériliser le dispositif de fixation interne est effectué en exposant le dispositif à un rayonnement.

24. La méthode de n'importe lesquelles des revendications 20 à 23, dans laquelle le dispositif de fixation interne ou le dispositif de fixation interne stérilisé est une vis, une broche, une tige, une plaque, une suture, une ancre de suture, une agrafe, un clip, ou un anneau.

25. La méthode de n'importe lesquelles des revendications 20 à 24, dans laquelle le carbonate de calcium a la structure cristalline de la calcite.

26. La méthode de n'importe lesquelles des revendications 20 à 25, dans laquelle le carbonate de calcium se présente sous la forme de particules de taille substantiellement uniforme.

27. La méthode de la revendication 26, dans laquelle une majorité des particules de carbonate de calcium ont une taille comprise entre 0,1 et 0,5 µm ; une taille comprise entre 0,5 et 2,5 µm ; une taille comprise entre 2,5 et 5,0 µm ; une taille comprise entre 5,0 et 7,5 µm ; ou une taille comprise entre 7,5 et 10,0 µm.

28. La méthode de n'importe lesquelles des revendications 20 à 25, dans laquelle le carbonate de calcium est présent sous forme de particules de carbonate de calcium dont la taille est comprise dans la gamme allant de 0,01 µm à 10,0 µm.

29. La méthode de n'importe lesquelles des revendications 20 à 28, dans laquelle les monomères d'acide lactique sont des monomères d'acide lactique en forme de L ou des monomères d'acide lactique en forme de D.

30. La méthode de n'importe lesquelles des revendications 20 à 28, dans laquelle les monomères d'acide lactique sont un mélange de monomères d'acide lactique en forme de L et en forme de D.

31. La méthode de n'importe lesquelles des revendications 20 à 30, dans laquelle le fait de combiner le carbonate de calcium et le copolymère comprend former un mélange substantiellement homogène.

32. La méthode de n'importe lesquelles des revendications 20 à 31, dans laquelle le carbonate de calcium constitue de 34 % à 36 % de la composition.

33. La méthode de la revendication 32, dans laquelle le carbonate de calcium constitue 35 % environ de la composition.

34. La méthode de n'importe lesquelles des revendications 20 à 31, dans laquelle le carbonate de calcium constitue de 36 % à 40 % de la composition.

35. La méthode de n'importe lesquelles des revendications 20 à 34, comprenant de plus le fait de fournir un agent thérapeutique et de combiner l'agent thérapeutique avec le copolymère et le carbonate de calcium.

36. Un dispositif de fixation interne réalisé par la méthode de n'importe lesquelles des revendications 20 à 35.

37. Un kit comprenant le dispositif de fixation interne de la revendication 36.

38. Un dispositif de fixation interne comprenant la composition biocompatible de n'importe lesquelles des revendications 1 à 19.

39. Le dispositif de fixation interne de la revendication 38, dans lequel le dispositif est une vis, une broche, une plaque, un clou, ou une ancre de suture.

40. Utilisation d'une composition ou d'un dispositif selon n'importe lesquelles des revendications 1 à 19 pour fabriquer un médicament destiné à réparer ou remodeler du tissu.

41. Utilisation d'une composition ou d'un dispositif selon n'importe lesquelles des revendications 1 à 19 pour fabriquer un médicament destiné au traitement de l'ostéomyélite.

42. Utilisation de la composition ou du dispositif selon la revendication 41, dans laquelle la composition ou le dispositif comprend un antibiotique, du carbonate de calcium et un copolymère formé à partir de monomères d'acide lactique et de monomères d'acide glycolique, dans laquelle le carbonate de calcium constitue plus de 30 % mais moins de 40 % du poids de la composition.

43. Utilisation d'une composition ou d'un dispositif selon n'importe lesquelles des revendications 1 à 19 pour fabriquer un médicament destiné au traitement du cancer des os.

44. Utilisation de la composition ou du dispositif selon la revendication 43, dans laquelle la composition ou le dispositif comprend un agent chimiothérapique, du carbonate de calcium et un copolymère formé à partir de monomères d'acide lactique et de monomères d'acide glycolique, dans laquelle le carbonate de calcium constitue plus de 30 % mais moins de 40 % du poids de la composition.
